(19)

Europäisches
Patentamt
European
Patent Office
Office européen
des brevets

(11) **EP 4 575 492 A1**

(12) **EUROPEAN PATENT APPLICATION**

(43) Date of publication:
**25.06.2025 Bulletin 2025/26**

(21) Application number: **23218049.7**

(22) Date of filing: **19.12.2023**

(51) International Patent Classification (IPC):
*G01N 33/18* *(2006.01)*

(52) Cooperative Patent Classification (CPC):
**G01N 33/188**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Faculty of civil engineering,
architecture and geodesy
University of Split
21000 Split (HR)**

(72) Inventor: **Andricevic, Roko
21000 Split (HR)**

(74) Representative: **Hadzija, Tomislav
BCI - Business Center International
1st Floor
Miramarska cesta 24
10000 Zagreb (HR)**

(54) **WATER QUALITY ASSESMENT METHOD**

(57)     Method for assessment of the quality of the water based on quantifying the uncertainty using the full probabilistic description for selected quality indicators at the reference and impacted site from the historical data record available for the study area. The method consist of the steps of selecting quality descriptors for status assessment, establishing of reference conditions under uncertainty, and development of the ecological quality ratio, wherein ecological quality ratio is presented through the concept of the environmental risk analysis as a probability of exceedance named the risk of deviation from the reference condition and it is evaluated through the distributional approach resulting in the probability distribution over five ecological classes.

Figure 1

EP 4 575 492 A1

## Description

### Technical field

The present invention relates to the field of measuring and testing and generally can be classified according to the IPC in class G01 N33/18 which specifically refers to investigating or analyzing water by determining the chemical or physical properties using specific methods.

### State of the art

The marine ecosystem is under strong anthropogenic pressures causing an environmental risk to coastal waters further emphasized by the climate change, which can influence the effects of these pressures and alter the hydrologic cycle. The coastal concentration of human population and human activities are changing the sea-land interface particularly in estuarine-coastal ecosystem.

The Water Framework Directive (WFD) that can be considered as the known state of the art, presented a framework for the ecological status assessment as a cooperative transnational effort in protecting all European waters including transitional and coastal waters. The WFD establishes a catchment-based approach addressing the ecological status focusing on three quality elements, primarily biological and supporting ones, physico-chemical and hydro morphological.

The challenges in the known state of the art and future development needs are imperative for future assessment improvements to increase the quality status effectiveness. The weaknesses of the current state of the art in this field could be summarized in a) "one-out, all-out" principle, b) the lack of different line of evidence delineating cause-effect relationship and c) the definition of reference conditions and degree of deviation from the reference.

The "one-out, all-out" principle results in assigning the lowest ecological status for the water body of any of the quality elements considered. This could be an applicable approach if different stressors are responsible for the deterioration of the individual quality elements. For most transitional and coastal water bodies, this might not be the case because some biological quality elements are sensitive to the same stressor and exhibit the high compounded uncertainty, causing over-precautionary results.

The current ecological and chemical status assessment known to the state the art incorporates multiple descriptors for a quality assessment scheme creating an extensive exercise for WFD implementation. In some other fields of environmental assessment, the concept of multiple lines of evidence analysis has been advanced, particularly in the Ecological Risk Assessment (ERA) procedures. Furthermore, regulatory institutions world-wide and particularly United States Environmental Protection Agency have recommended retrospective risk assessment with stepwise strategies to evaluate ecosystem health and support the site-specific remediation measures. The WFD could benefit from the ERA "philosophy", which integrates multiple lines of evidence between stressor and effect in the risk characterization, using the quantification of uncertainty as a driving force for the decision making, this cause-effect relationship, with the assessment of uncertainty, could provide a way to focus on key site-specific descriptors which are determinants for assessing the ecological and chemical status of the specific water body.

In addition, very common misinterpretations are due to the lack of clarity of definitions related to the reference condition, the degree of deviation from the reference condition, and defining boundaries between ecological classes.

The inter-calibration exercise will always have its challenges and exemptions due to the different methods used and priorities regarding the national development needs, which can compromise the overall effectiveness of the WFD. The reference condition selection and boundaries between ecological classes will always be subject to the aleatory uncertainty as natural randomness of quality descriptors and particularly from the epistemic uncertainty due to the space and time limited data (acquired through monitoring), which will be even more pronounced in the future due to the climate change impact.

Instead of dealing with statistical measures in terms of geometric means and/or some quantiles of quality descriptors, the method from the current patent application proposes an alternative approach, which is based on quantifying the uncertainty using the full probabilistic description for selected quality indicators at the reference and impacted site from the available data set. The modified concept of environmental risk analysis is used to define the ecological quality ratio (EQR) as a risk of deviation from the reference condition sensu WFD. The resulting ecological quality ratio is derived through the distributional approach for quality indicators at the reference and impacted site, enabling to estimate the ecological quality ratio probability of being in each of five quality classes for a considered water body. The ability to estimate the likelihood of being in five quality classes, enables decision makers, responsible for the WFD implementation, to select the Program of Measures, which will be specifically tailored to reduce the EQR probability of being below a good quality class, which is the ultimate goal of the WFD implementation. The classification criteria are based on the trophic status and related coastal water quality conditions. The developed method is presented in the case study of coastal and transitional water bodies in the Central Eastern Adriatic Sea, based on the comprehensive knowledge of local hydrodynamics and the cause-effect relationship.

The water quality assessment required by EU regulations is primarily focused on the integration of a range of descriptors relating to biological quality elements (BQE), together with physico-chemical and hydro morphological as supporting quality elements. Regardless of water body type, phytoplankton is one of the most desired quality elements including its attributes like bloom frequency, composition, and abundance However, the feasibility and difficulties in assessing all possible attributes for the phytoplankton community resulted in the use of chlorophyll $\alpha$ as a status indicator because of its easier sampling techniques with new technologies (spectrophotometric and fluorometric methods) and availability of data sets for assessment procedures. The chlorophyll $\alpha$(Chl-$\alpha$) together with oxygen absolute deviation from the saturation (aDO), the dissolved inorganic nitrogen (DIN) and phosphorus as Total Phosphorus (TP) or phosphate ($PO_4$), constitute the composite trophic status index (TRIX). The TRIX index is not fully consistent with the WFD because of combining the pressure (DIN and TP) and impact (Chl $\alpha$ and aDO) in the integrated fashion. However, the trophic scale described by the TRIX index in marine waters can be used as a control variable in the quality assessment.

The enrichment of nutrients, especially compounds of nitrogen and phosphorous, in a marine environment is a major factor contributing to the failure to achieve good ecological status. Thus, the new technology presented in the invention from the current patent application assigns the abiotic component, Log[DIN·$PO_4$], as a pressure indicator causing a potential impact on the quality of marine waters. This component can be considered a general physicochemical quality element supporting BQE for ecological status assessment. Consequently, the effect of this pressure is manifested in the productivity component Log[Chl$\alpha$·aDO], which is a status indicator for the phytoplankton presence and produced biomass in marine waters. This, spatial and temporal variability analysis of productivity and abiotic indicators is used for the ecological quality assessment for transitional and coastal water bodies in this new technology.

The selected indicators for pressure and impact are subject to complex biological, chemical, and mixing processes exhibiting strong spatial and temporal variability. The evidence of nutrient enrichment and resulting production is required to meet some acceptable standards for a good classification in coastal waters according to the WFD. Finding correlations between nutrient concentration and biology can be very challenging due to the spatial variability and different patterns of nutrient limitations in marine waters. Many EU member states reported different methods for nutrient and biomass production criteria and corresponding thresholds. These methods are often related to the use of different statistical measures, water quality modelling, regression analysis, and/or expert judgment, depending on the size and quality of available data. What is common in all these methods is the unavoidable uncertainty present in selecting reference conditions and boundaries between ecological classes. For marine waters, this is a particular challenge when intercalibrated BQE is required as a starting point for defining the nutrient boundaries.

The monitoring programs usually available for the ecological status assessment are sampling sessions representing snapshots of water quality parameters in space and time. Thus, it is reasonable to treat quality elements from data as random variables or even in some cases (if enough spatial and temporal coverage is available, e.g. using airborne or satellite data) a random field with a certain spatial and temporal structure. In this new method, the selected descriptors will be treated as random variables.

According to the WFD for the ecological status assessment, the type-specific Reference Conditions (RC) represent biological quality elements at high status with no, or very minor, effect from the anthropogenic pressure. For the chemical status assessment, the common approach is based on selecting a threshold value for physicochemical quality elements based on national water quality standards. In most quality assessments we are faced with sparse spatial and temporal measurements (or even with the lack of data for some water bodies) exhibiting high natural variability, which hinders the precise establishment of reference conditions as pristine-like conditions, particularly the acceptable deviation. This has been demonstrated as an important problem in assessing the ecological status described with the categorical classification (high, good, moderate, poor, and bad) in the WFD.

The new method described in the present application reveal consideration of the RC for productivity and abiotic indicators as a random variable with a distribution estimated from the available monitoring data.

Some of the challenges in the current state of the art are explained above when improvements to the current WFD were describe. Additionally, despite the overall understanding of the applicant, the following patent applications are considered as the closest to the prior art.

CN114004552A - "Underground water quality risk assessment method system" that relates to the technical field of water quality detection, and particularly discloses an underground water quality risk assessment method that receives input information of a user, determines an assessment area according to the input information; generates a three-dimensional model, performs region division on the three-dimensional model to obtain sub-regions containing influence values, calculates a water quality risk value of each sampling point in the evaluation area according to the influence value, and compares the water quality risk value with a preset risk threshold value.

AU2021106282A4 - "A method for quickly tracing the source of water pollution emergencies in a river basin" that relates to a method for quickly tracing the source of water pollution emergencies in a river basin, which includes the steps of obtaining the distribution of potential pollution source areas; monitoring water quality components in the potential pollution source areas; selecting pollutants, which's concentration is higher than allowable concentration standard or higher than 3 times the background value, used as the characteristic pollution factors; build a tracing model; calculate the pollution source probability of the risk-sensitive area in real time; count the pollutants concentration and contribution to the concentration of pollutants from the sewage drainage industries list in the risk-sensitive area.

CN1 08876226A - Water quality assessment method and device that comprising the steps of setting an evaluation index ci, wherein the evaluation index includes at least two items and includes a chlorophyll concentration; constructing a water quality assessment model T; acquiring a concentration calculating model corresponding to each evaluation index; according to the concentration calculating model and a clustering algorithm, acquiring the final concentration of each evaluation index; according to the final concentration of each evaluation index, grading a water body, wherein water body grading according to the final concentration of each evaluation index includes calculating an integration evaluation set according to the final concentration of each evaluation index and the water quality assessment model; and determining a water body grade according to the position of a maximum value in the integration evaluation set.

In the all above mentioned cases described above regardless of what kind of water quality assessment method is applied there is no mention of using the productivity and abiotic indicators as metrics for transitional and coastal waters that allow to assess the water quality status. Hereby, in the current patent application, the proposed invention utilizes the logarithmic nature of the product of variables to describe the selected metrics with a full distributional description using geometric mean values and/or some quantiles for selected metrics

Additionally, the ecological quality ratio proposed in the current application is a measure of the likelihood of exceeding the risk of deviation (RoD) and the use of RoD is a novel aspect of the presented application that allows for the EQR to be described by a distribution with five ecological classes, as prescribed in the WFD.

The presented new method could be viewed as a retrospective ecological quality assessment used to identify the RoD from stressors already present in the coastal waters. The ability to estimate the probability that impacted site (or a water body under assessment) could be in any of five quality classes enables decision-makers, responsible for the WFD implementation, to select the Program of Measures, which will be specifically tailored to reduce the ecological quality ratio probability of being below a good quality class.

**Brief description of the drawings**

Along with the summary and the detailed description as well for a better understanding of the features of the invention, in accordance with the explanation of the practical implementation of the invention, the following figures are presented:

**Figure 1** - Histogram with estimated distribution for Log[Chl$\alpha$($\mu$g/l)$\cdot\alpha$DO] and Log[DIN($\mu$g/l). PO$_4$($\mu$g/l)] in the study area,

**Figure 2** - Relationship between TRIX trophic index to productivity (adjusted R$^2$ = 0.92) and abiotic component (adjusted R$^2$ = 0.97),

**Figure 3** - Transitional and coastal water bodies in the Kaštela Bay area with corresponding sampling stations,

**Figure 4** - Comparison of marginal CDFs for productivity and abiotic indicators at station FP-P9a (Site) with distributions estimated for the reference site (Ref),

**Figure 5** - The EQR probabilities for productivity and abiotic indicator for five quality classes for impacted site of the station FP-P9a within the transitional water body P1_2.

**Figure 6a** - An inverse relationship between productivity indicator and river Jadro flow rate (R$^2$=0.49, P-value 5.5 $\times$ 10$^{-5}$),

**Figure 6b** - An inverse relationship between nitrate NO$_3$ and Chl$\alpha$ (R$^2$=0.38, P-value 6.8$\times$10$^{-4}$) for the station FP-P9a.

**Figure 7** - The EQR probabilities for five quality classes for the transitional water body P2_2 at stations FP-P10 and FP-P10a,

**Figure 8** - The EQR probabilities for five quality classes for the coastal water body 0313-KASP at stations FP-O16 and FP-O16b.

**Summary of the invention**

A method for the ecological status assessment based on quantifying the uncertainty of selected quality descriptors through the probabilistic description is hereby presented. The assessment method is based on two metrics: the logarithm of multiplied chlorophyll $\alpha$ with the oxygen absolute deviation from the saturation as a productivity indicator and the logarithm of multiplied dissolved inorganic nitrogen and phosphorus as supporting metric in the form of an abiotic indicator. All four quality parameters are commonly measured in marine monitoring programs and they represent the easily attainable data required by the WFD.

Instead of using geometric mean values and/or some quantiles for selected metrics, this method utilizes the logarithmic nature of the product of variables to describe the selected metrics with a full distributional description. The ecological quality ratio (EQR) is presented through the concept of the environmental risk analysis as a probability of exceedance named the risk of deviation from the reference condition. The EQR is evaluated through the distributional approach resulting in the probability distribution over five ecological classes. The classification boundaries for each selected metric is determined based on the correlation between the TRIX index scale, which relates the trophic status and corresponding coastal water quality conditions. The ability to estimate the likelihood of being in five quality classes, for each water body, allows decision-makers to better grasp the actual quality status in terms of how likely the water body can be designated to the good ecological class, which is the ultimate goal in the WFD implementation process.

Using the productivity and abiotic indicators as two metrics for transitional waters provides an opportunity for analyzing the relationship between river flow fluctuation and water quality parameters. This enables the measurement of estuarine responses to the freshwater impact on the biological community. In the case of the river Jadro estuary, the biomass productivity is limited during high flows when the flushing rate exceeds the phytoplankton growth rate. The relatively fast exchange flow dynamics create different timing between the process of converting dissolved nutrients into the new biomass and the freshwater residence time. In the lower part of the estuary, the increased mixing between freshwater discharge and landward inflow of the seawater mass at the bottom is generating an additional vertical and lateral mixing resulting in shifting the EQR distribution towards the higher quality classes.

This method presented a concept of the risk of deviation from the reference site as an alternative water quality assessment method, based on quantifying the uncertainty using the full probabilistic description for selected quality indicators at the reference and impacted site from the historical data record available for the study area. It is based on the site specific hydrodynamics of the river Jadro estuary, local terrestrial loadings and very mild tidal effects. The calculation procedure is semi-analytical and a large number of sites and available data could be analyzed relatively fast.

**Detailed description of the invention**

The water quality assessment method starts with process of selecting quality descriptors for ecological status assessment.

The chlorophyll $\alpha$ (Chl-$\alpha$) together with oxygen absolute deviation from the saturation (aDO), the dissolved inorganic nitrogen (DIN) and phosphorus as Total Phosphorus (TP) or phosphate (PO$_4$), constitute the composite trophic status index (TRIX). The TRIX index is not fully consistent with the WFD because of combining the pressure (DIN and TP) and impact (Chl$\alpha$ and aDO) in the integrated fashion. However, the trophic scale described by the TRIX index in marine waters can be used as a control variable in the water quality assessment.

The enrichment of nutrients, especially compounds of nitrogen and phosphorous, in a marine environment is a major factor contributing to the failure to achieve good ecological status.

This method assigns the abiotic component, Log[DIN·PO$_4$], as a pressure indicator causing a potential impact on the quality of marine waters. This component can be considered a general physicochemical quality element supporting biological quality elements for ecological status assessment. Consequently, the effect of this pressure is manifested in the productivity component Log [Chl$\alpha$·aDO], which is a status indicator for the phytoplankton presence and produced biomass in marine waters. This, spatial and temporal variability analysis of productivity and abiotic indicators is used for the ecological quality assessment for transitional and coastal water bodies in this technology.

The selected indicators for pressure and impact are subject to complex biological, chemical, and mixing processes exhibiting strong spatial and temporal variability. The evidence of nutrient enrichment and resulting production is required to meet some acceptable standards for a good classification in coastal waters according to the WFD. Finding correlations between nutrient concentration and biology can be very challenging due to the spatial variability and different patterns of nutrient limitations in marine waters. Many EU member states reported different methods for nutrient and biomass production criteria and corresponding thresholds. These methods are often related to the use of different statistical measures, water quality modelling, regression analysis, and/or expert judgment, depending on the size and quality of available data. What is common in all these methods is the unavoidable uncertainty present in selecting reference conditions and boundaries between ecological classes. For marine waters, this is a particular challenge when intercalibrated BQE is required as a starting point for defining the nutrient boundaries.

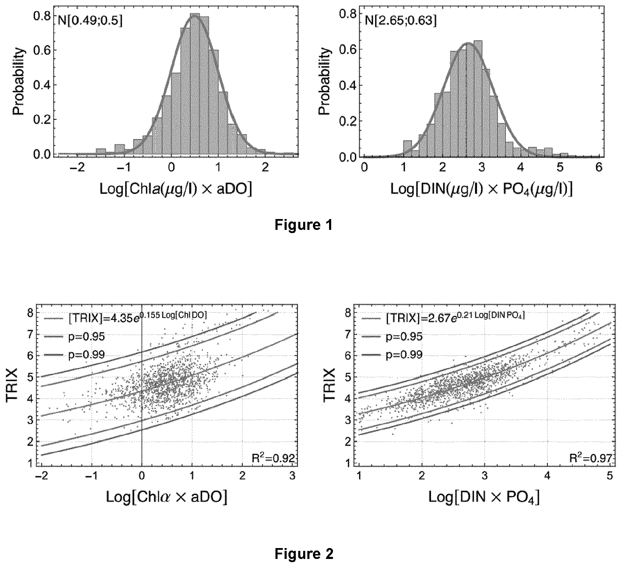

The monitoring programs usually available for the ecological status assessment are sampling sessions representing snapshots of water quality parameters in space and time. Thus, it is reasonable to treat quality elements from data as random variables or even in some cases (if enough spatial and temporal coverage is available, e.g. using airborne or satellite data) a random field with a certain spatial and temporal structure. In this new method, the selected descriptors will be treated as random variables. For example, the Figure 1 presents the histogram with estimated probability distribution function (PDF) for the productivity and abiotic indicator using the entire data set of 13 monitoring stations in the area of Kaštela Bay and Brač canal near the city of Split, Croatia, during the period 2012-2019 (N = 1414 samples).

The logarithmic transformation of multiplied water quality parameters (proposed above) tends to quickly approach the normality or close to normality behavior in their statistical distributions. The range of values indicates the quality indicators spread found in the spatial (monitoring stations available in the coastal area) and a temporal extent (historic monitoring record) of available data.

The next step in the method of the water quality assessment is the <u>establishing the reference conditions under uncertainty.</u> The reference conditions for productivity and abiotic indicators are considered as a random variable with a distribution estimated from the available monitoring data. The relationship between the TRIX trophic scale with each of the four components for transitional and coastal water bodies can be often affected by different processes like surface runoff, river discharges, spatial and temporal variability in loading, and hydrodynamics driven by atmospheric conditions and climate change impacts. However, when relating the TRIX index to the productivity and abiotic indicators separately a clear trend can be found. The following Figure 2 presents this relationship with two confidence levels for 13 sampling stations considered in the study area.

The method of the water quality assessment further continues with the establishing <u>of the ecological quality ratio.</u>

For the above selected quality indicators, the ecological quality ratio is calculated with a simple equation:

$$EQR_i = R_i / S_i \qquad (1)$$

where $R_i$ and $S_i$ denote the quality indicator i at the reference site and impacted site, respectively. Since both quality indicators are treated as random variables, the resulting EQR also follows the probability distribution. For estimating how the EQR is distributed over the categorical classification, the next step is to define classification criteria for chosen quality indicators.

Following the water quality condition described with the TRIX index, the method used selection of TRIX units to define classification boundaries. The critical boundary is the good/moderate (G/M) boundary, which identifies whether the analyzed water body requires a program of measures in case of failing the good status. The G/M boundary was set to correspond to the TRIX = 5 reflecting the transition from mesotrophic to eutrophic conditions in coastal waters. The presence of quality indicators, presented above are further identified and quantified. The cause-effect relationship between the stressor and trophic status of transitional and coastal water bodies is analyzed and finally, the risk characterization is performed considering the distribution of EQR as a measure of the relative deviation from the reference conditions (RC) or simply the risk of deviation (RoD).

RoD describes the process which evaluates the likelihood that adverse environmental impacts on the water body are due to the presence of identified stressors and impact in the water body. This probability of exceedance is defined as a measure of the RoD for $i^{th}$ quality indicator as $P(Ri \leq Si)$, where $P(\cdot)$ stands for the probability. For statistically independent quality indicators at reference and impacted site, the risk of deviation can be evaluated as:

$$P\left(\frac{R_i}{S_i} \leq 1\right) = \int_{S_L}^{S_U} \int_{R_L}^{s} f_R(r) f_S(s) \, dr \, ds \qquad (2)$$

where $f_R$ and $f_S$ are PDFs for the $i^{the}$ quality indicator at the reference and impacted site, respectively, and $(S_L, S_U)$ and $(R_L, R_U)$ are the lower and upper bounds for the quality indicator at the impacted site and reference site, respectively. By solving the inside integral in above expression, the probability of deviation can be further reduced to:

$$P\left(\frac{R_i}{S_i} \leq 1\right) = \int_{S_L}^{S_U} F_R(s) f_S(s) \, ds \qquad (3)$$

where FR(s) is the marginal cumulative distribution function (CDF) of the random quality indicator at the reference site. Using the classification boundaries derived from Figure 2, for example, the probability that the impacted site will be within a good class can be computed from:

$$P\left(\frac{G}{M} < \frac{R_i}{S_i} \leq \frac{H}{G}\right) = \int_{G/M}^{H/G} F_R(s) f_S(s) \, ds \qquad (4)$$

where limits of integration are boundaries of the good ecological quality class. By the same token, one can compute the probability that the impacted site could be in any of five quality classes. If one is interested only in probabilities of being in a good and higher class or failing to be in a good class (e.g., in the case of chemical quality assessment with G/M boundary representing a threshold) the following expression can be used:

$$P\left(\frac{R_i}{S_i} \leq Good\right) = \int_{s_L}^{G/M} F_R(s)f_S(s)ds \qquad (5)$$

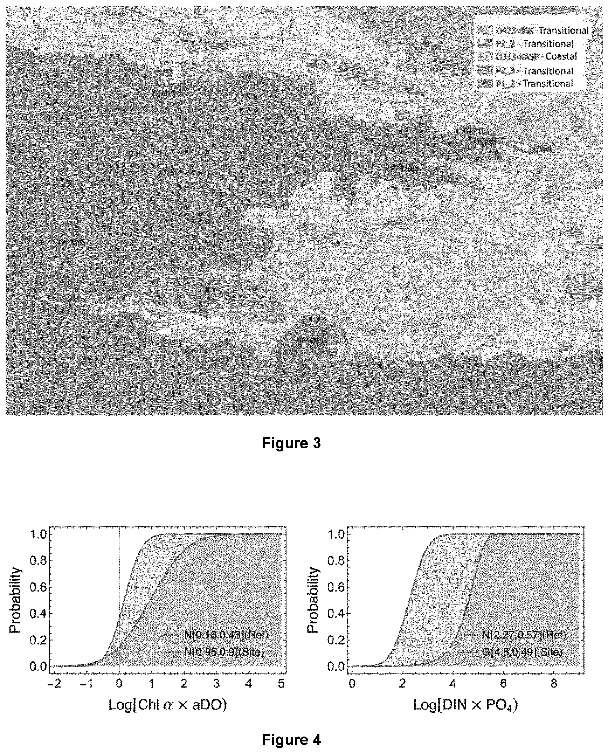

The above explained method for water quality assessment is used for evaluation of the ecological status for transitional and coastal water bodies in the Kaštela bay area. Figure 3 depicts two transitional water bodies for the river Jadro estuary and one coastal water body for the Kaštela Bay identified by the Croatian Water Agency that will be assessed using the proposed method. In the following, the original labels from figure 3 will be used for each sampling station and the corresponding water body. Table 1. summarizes the estimated PDFs for each sampling station in the Kaštela Bay based on the available data set from 2012 to 2019. The estimation procedure was performed in the Mathematica package using the Cramer-von Mises (CVM) goodness-of-fit test with a corresponding P-value. Besides the Normal distribution, most often found, selected indicators at some stations exhibit a slight skewness in the sampling data. In that case, they are described with the Gumbel distribution denoted with G[$\alpha$, $\beta$] in Table 1, where $\alpha$ and $\beta$ are location and scale parameters, respectively.

**Table 1**

| Estimated distributions for quality indicators at sampling stations in transitional and coastal water bodies of Kaštela Bay with Cramer-von Mises test and the corresponding P-value. | | | | |
|---|---|---|---|---|
| | *Log[Chl$\alpha$·aDO]* | *CVM P-value* | *Log[DIN·PC$_4$]* | *CVM P-value* |
| FP-P9a | N[0.95,0.91] | 0.98 | G[4.8,0.49] | 0.94 |
| FP-P10 | N[0.99,0.43] | 0.3 | N[3.67,0.73] | 0.5 |
| FP-P10a | G[1.04,0.39] | 0.53 | N[2.85,0.5] | 0.6 |
| FP-O16 | G[0.83,0.41] | 0.86 | N[2.6,0.55] | 0.93 |
| FP-O16b | G[0.87,0.39] | 0.92 | N[2.71,0.55] | 0.56 |

Water quality assessment for transitional water body P1 2 in the upper Jadro estuary

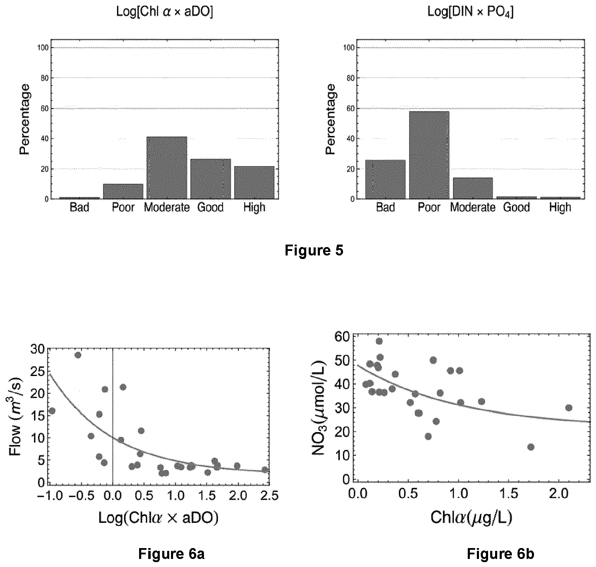

The transitional water body P1_2 represents the upper part of the Jadro estuary with a total length of 1.5 km (Figure 3) and contains the station FP-P9a. Using the historical record of measurements, the estimated marginal CDFs for productivity and abiotic quality indicators for station FP-P9a and designated reference site distributions are jointly presented in Figure 4. The location parameter and scale parameter show a certain deviation from the considered reference site distribution, which is more pronounced for the abiotic indicator than for Log[Chl$\alpha$·aDO], Using Formula (3), the EQR, defined as a risk of deviation from the reference site, is computed and displayed in Figure 5. The abiotic indicator shows a larger deviation from the reference site and the risk of deviation is predominantly distributed between three lower classes, which require measures to be taken sensu WFD. This should not be surprising for the upper transitional water body of the river Jadro estuary knowing that the major stressor is the wastewater discharge not only through the network of torrents and outlets but also continuously discharged from the upper horizons of a hydrological catchment. This source of terrestrial loadings clearly presents the strong anthropogenic pressure on transitional water bodies. On the other hand, the risk of deviation for the productivity indicator is centered around the moderate quality class and slightly leaning towards the upper classes. This result might indicate some constraints in the phytoplankton production capabilities present within the upper transitional water body P1_2. The station FP-P9a sampling data show that variability of water quality parameters is closely related to fluctuations of river flow. The circulation in the estuary described with the exchange flow pattern (net seaward flow and its counterpart as a density-driven flow landward) creates estuarine responses from biological processes to the wide range of river flows.

This impact from the hydro-climatic forcing can be seen in Figure 6a, displaying an inverse relationship between the river Jadro flow and productivity indicator *Log[Chl$\alpha$·aDO]*. For analyzed transitional water body P1_2 the low primary production of biomass is limited in the accumulation during high flows when flushing rate exceeds phytoplankton growth rate. Figure 6b also shows an inverse relationship between nitrate ($NO_3$), which constitutes around 85% of DIN, and Chl$\alpha$ indicating the suppression of biomass growth with the higher dissolved inorganic nitrogen presence. This characteristic was often tied with the high ammonium ($NH_4$) presence as inhibiting factor for phytoplankton utilization of the nitrogen supply, calling this phenomenon an ammonium-suppression hypothesis. This was supported by measurements elsewhere showing the reduced nitrate ($NO_3$) uptake when $NH_4$ exceed about 4 $\mu$mol/L. However, at station FP-P9a the ammonium does not exceed 4 $\mu$mol/L except in a single sample in 2014. Thus, in our case study this inverse relationship between ($NO_3$) and Chl$\alpha$ could be explained in different timing between the process of converting dissolved nutrients into the new biomass and the freshwater residence time. Thus, the negative correlation between nitrate and chlorophyll a may not be due to the growth suppression, but rather to the nutrient fast uptake during blooms when chlorophyll concentration increases.

Water quality assessment for transitional water body P2 2 - lower Jadro estuary

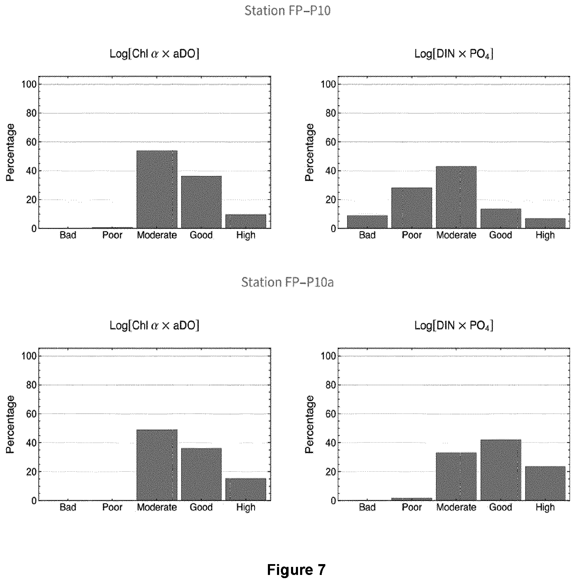

The transitional water body P2_2 is located in the lower part of the river Jadro estuary and contains two sampling stations, FP-P10 and FP-P10a, which will be analyzed individually for the ecological quality assessment. Figure 7 displays the EQR distribution over water quality classes for both stations. The station FP-P10 is located along the river discharge path-line and shows a slight improvement in the productivity indicator from the station FP-P9a, with the EQR probability distributed within the upper three quality classes.

The abiotic indicator, on the other hand, shows the quality condition centered at the moderate class with still 30% chance of being assigned to the poor class. Comparing the abiotic indicator quality ratio from the station FP-P9a (Figure 5), it is clear that in the lower part of the estuary there is an improvement in the quality condition. This quality class improvement for this transitional water body is due to the increased mixing between freshwater discharge and landward inflow of seawater mass at the bottom. This exchange flow is generating the additional vertical mixing between fresh and saltwater masses and creates additional lateral spreading of a nutrient load coming from the river Jadro discharge.

The station FP-P10a is located off the river Jadro flow direction, very close to the coastline (Figure 3). The productivity indicator $Log[Chl\alpha \cdot aDO]$ has similar EQR distribution compared to FP-P10 indicating the similar biomass growth rate. However, the abiotic indicator $Log[DIN \cdot PQ_4]$ shows the EQR distribution further shifting towards the good and high-quality condition as a result of increased lateral mixing processes taking place in the wider estuary area. If one looks at the probability for assigning the abiotic indicator to good or fail quality status only, there is approximately 40% chance for failing and 60% chance for assigning the good condition. Historical data used in this study shows the strong influence from the nutrient loading to the water quality assessment for transitional water bodies in the river Jadro estuary. This loading predominantly originates from the municipal wastewater discharge not only through the topographic river catchment but also from the hydrologic one, which drains the surrounding inland region with agricultural activities and no public sewage system yet in place. This loading is identified as a key stressor from the line of evidence analysis and its influence on the water quality is demonstrated with EQR distribution leaning towards less than good quality conditions in transitional water bodies P1_2 and P2_2.

3.4.3. Quality assessment for coastal water body 0313-KASP in the Kaštela Bay

The coastal water body O313-KASP comprises most of the Kaštela Bay area and it is represented with stations FP-O16 and FP-O16b. The influence from the river Jadro estuary is diminishing which can be seen from the location and scale parameters of these stations' estimated distributions in Table 1. In addition, the western part of the Kaštela Bay is connected to the Brač canal with increased hydrodynamic mixing due to the sea currents and surface waves.

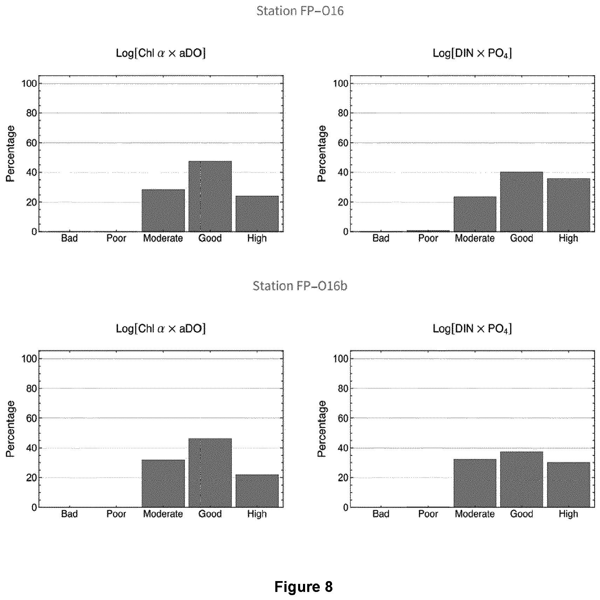

The mean value of the abiotic indicator is only slightly increased from the reference site with a similar scale parameter. The resulting EQR is leaning towards the good and high classes for productivity and abiotic indicators for both sampling stations (Figure 8). The loading of nutrients, which originated from the river Jadro discharges are relatively quickly mixed with the seawater of the Kaštela Bay resulting in the lower mean value of the abiotic indicator. However, this reduction in the nutrient level is not directly followed by the production indicator. The time for the production of phytoplankton is now sufficient for the biomass to become visible through the $Chl\alpha$ concentration levels on average. The ecological quality assessment for the coastal water body

O313-KASP could be estimated to be closer to good condition than otherwise. The EQR percentages for each quality class can also be viewed as percentages of samples from the impacted site having the quality condition of that class for selected quality indicators.

**Claims**

**1.** Method for assessment of the quality of the water based on quantifying the uncertainty using the full probabilistic description for selected quality indicators at the reference and impacted site from the historical data record available for the study area, **characterized that** consist of the following steps of:

- selecting quality descriptors for status assessment,
- establishing of reference conditions under uncertainty, and
- development of the ecological quality ratio,

wherein ecological quality ratio is presented through the concept of the environmental risk analysis as a probability of exceedance named the risk of deviation from the reference condition and it is evaluated through the distributional approach resulting in the probability distribution over five ecological classes.

2. The method in accordance to the patent claim 1, **characterized that** the step of selecting quality descriptors consist of utilizing the productivity and abiotic indicators as metrics for transitional and coastal waters that allows assessing water quality status wherein the logarithm of multiplied chlorophyll a with the oxygen absolute deviation from the saturation Log [Chl$\alpha$·aDO] represent the productivity indicator and the logarithm of multiplied dissolved inorganic nitrogen and phosphorus Log[DIN·PO$_4$], represent the supporting metric in the form of an abiotic indicator in which all four quality parameters are commonly measured in marine monitoring programs.

3. The method in accordance to the patent claim 1, **characterized that** the step of establishing of reference conditions under uncertainty wherein the said reference condition for productivity and abiotic indicators are considered as a random variable with a distribution estimated from the available monitoring data.

4. The method in accordance to the patent claim 1, **characterized that** the step of development of the ecological quality ratio (EQR) for the selected quality indicators consist of using the following equation:

$$EQR_i = R_i / S_i \qquad (1)$$

where:

$R_i$ denote the quality indicator i at the reference site, and
$S_i$ denote the quality indicator i at the impacted site,

and both quality indicators are treated as random variables, resulting that EQR also follows the probability distribution.

5. The method in accordance to the patent claim 4, **characterized that** the step of development of the ecological quality ratio (EQR) further include the step of defining classification criteria for chosen quality indicators in order to estimate how the EQR is distributed over the categorical classification whereby selection of TRIX units are used to define classification boundaries in which the critical boundary is the good/moderate (G/M) boundary, which identifies whether the analyzed water body requires a program of measures in case of failing the good status.

6. The method in accordance to the patent claim 5, **characterized that** the G/M boundary is set to correspond to the TRIX = 5 reflecting the transition from mesotrophic to eutrophic conditions in coastal waters and the presence of quality indicators, presented above are further identified and quantified wherein the cause-effect relationship between the stressor and trophic status of transitional and coastal water bodies is analyzed and the risk characterization is performed considering the distribution of EQR as a measure of the relative deviation from the reference conditions (RC) or the risk of deviation (RoD).

7. The method in accordance to the patent claim 6, **characterized that** RoD describes the process which evaluates the likelihood that adverse environmental impacts on the water body are due to the presence of identified stressors and impact in the water body wherein this probability of exceedance is defined as a measure of the RoD for *ith* quality indicator as P($R_i \leq S_i$), where P(.) stands for the probability, where for example for statistically independent quality indicators at reference and impacted site, the risk of deviation can be evaluated as:

$$P\left(\frac{R_i}{S_i} \leq 1\right) = \int_{S_L}^{S_U} \int_{R_L}^{s} f_R(r) f_S(s) dr ds \qquad (2)$$

where:

$f_R$ and $f_S$ are PDFs for the $i^{the}$ quality indicator at the reference and impacted site, respectively,
and ($S_L$, $S_U$) and ($R_L$, $R_U$) are the lower and upper bounds for the quality indicator at the impacted site and reference site, respectively
and additionally by solving the inside integral in above expression, the probability of deviation can be further reduced to:

$$P\left(\frac{R_i}{S_i} \leq 1\right) = \int_{S_L}^{S_U} F_R(s)f_S(s)ds \qquad (3)$$

where $F_R(s)$ is the marginal cumulative distribution function (CDF) of the random quality indicator at the reference site.

8.  The method in accordance to the patent claim 7, characterized that using the classification boundaries, for example, the probability that the impacted site will be within a good class can be computed from the following formula:

$$P\left(\frac{G}{M} < \frac{R_i}{S_i} \leq \frac{H}{G}\right) = \int_{G/M}^{H/G} F_R(s)f_S(s)ds \qquad (4)$$

where limits of integration are boundaries of the good ecological quality class, consequently, by the same token, it can be compute the probability that the impacted site could be in any of five quality classes, additionally for the purpose of estimating the probabilities of being in a good and higher class or failing to be in a good class (e.g., in the case of chemical quality assessment with G/M boundary representing a threshold) the following formula is used:

$$P\left(\frac{R_i}{S_i} \leq Good\right) = \int_{S_L}^{G/M} F_R(s)f_S(s)ds \qquad (5)$$

Figure 1

Figure 2

Figure 3

Figure 4

Log[Chl $\alpha$ × aDO]

Log[DIN × PO$_4$]

**Figure 5**

**Figure 6a**

**Figure 6b**

Figure 7

Station FP-O16

Log[Chl $\alpha$ × aDO]

Log[DIN × PO$_4$]

Station FP-O16b

Log[Chl $\alpha$ × aDO]

Log[DIN × PO$_4$]

**Figure 8**

| | Europäisches Patentamt<br>European Patent Office<br>Office européen des brevets | **EUROPEAN SEARCH REPORT** | **Application Number**<br>**EP 23 21 8049** |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CARSTENSEN ET AL: "Statistical principles for ecological status classification of Water Framework Directive monitoring data", MARINE POLLUTION BULLETIN, OXFORD, GB, vol. 55, no. 1-6, 23 November 2006 (2006-11-23), pages 3-15, XP005778106, ISSN: 0025-326X, DOI: 10.1016/J.MARPOLBUL.2006.08.016 * "5.3.3 Assessment of ecological status" * | 1-8 | INV.<br>G01N33/18 |
| A | NEWTON ALICE ET AL: "An overview of ecological status, vulnerability and future perspectives of European large shallow, semi-enclosed coastal systems, lagoons and transitional waters", ESTUARINE, COASTAL AND SHELF SCIENCE, NEW YORK, NY, US, vol. 140, 14 June 2013 (2013-06-14), pages 95-122, XP028831542, ISSN: 0272-7714, DOI: 10.1016/J.ECSS.2013.05.023 * 3. Statistical classification * | 3,7 | |

-/--

TECHNICAL FIELDS
SEARCHED (IPC)

G01N

The present search report has been drawn up for all claims

| Place of search | Date of completion of the search | Examiner |
|---|---|---|
| Munich | 21 March 2024 | Chabot, Pedro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................................
& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 23 21 8049

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DOS SANTOS SÁ ANA KAROLINE DUARTE ET AL: "Multiple stressors influencing the general eutrophication status of transitional waters of the Brazilian tropical coast: An approach utilizing the pressure, state, and response (PSR) framework", JOURNAL OF SEA RESEARCH, ELSEVIER, AMSTERDAM, NL, vol. 189, 24 September 2022 (2022-09-24), XP087205127, ISSN: 1385-1101, DOI: 10.1016/J.SEARES.2022.102282 [retrieved on 2022-09-24] * 2.5 Trophic index (TRIX) * | 5,6 | |
| A | MICHELLE DEVLIN ET AL: "Comparison of five methods for assessing impacts of nutrient enrichment using estuarine case studies", BIOGEOCHEMISTRY, KLUWER ACADEMIC PUBLISHERS, DO, vol. 106, no. 2, 22 March 2011 (2011-03-22), pages 177-205, XP019979729, ISSN: 1573-515X, DOI: 10.1007/S10533-011-9588-9 * page 192 - page 194 * | 2,4 | TECHNICAL FIELDS SEARCHED (IPC) |

The present search report has been drawn up for all claims

| Place of search | Date of completion of the search | Examiner |
|---|---|---|
| Munich | 21 March 2024 | Chabot, Pedro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 114004552 A
- AU 2021106282 A4

- CN 108876226 A